# EUROPÄISCHE PATENTSCHRIFT

(11) **EP 2 926 840 B1**
(45) Veröffentlichungstag und Bekanntmachung des Hinweises auf die Patenterteilung: **09.05.2018**
(21) Anmeldenummer: 15157296.3
(22) Anmeldetag: 03.03.2015
(51) Int. Cl.: A61L 27/36

(54) **Verfahren zur Aufbereitung von biologischem Gewebe zur trockenen Verwendung in einem Implantat**
Method for the treatment of biological tissue for dry use in an implant
Procédé de traitement de tissu biologique pour une utilisation à sec dans un implant

(30) Priorität: 02.04.2014 EP 14163120
(43) Veröffentlichungstag der Anmeldung: 07.10.2015
(73) Patentinhaber: Biotronik AG, 8180 Bülach (CH)
(72) Erfinder: Lehenberger, Nina, 91052 Erlangen (DE); Rzany, Alexander, 90449 Nürnberg (DE); Erdbrügger, Wilhelm, 78467 Konstanz (DE)
(74) Vertreter: Galander, Marcus

(56) Entgegenhaltungen:
- WO-A1-99/66967
- US-A- 4 357 274
- US-A1- 2010 030 340

## Beschreibung

Die vorliegende Erfindung betrifft ein Verfahren zur Aufbereitung von biologischem Gewebe für die Verwendung als Bestandteil eines Implantats, insbesondere zur Verwendung als Bestandteil einer Herzklappenprothese, sowie ein Implantat, welches derart aufbereitetes biologisches Gewebe im getrockneten Zustand enthält.

Die Erfindung wird im Folgenden am Beispiel eines Verfahrens zur Aufbereitung von Gewebe zur Verwendung für eine künstliche Herzklappe beschrieben. Die vorliegende Erfindung ist zwar zur Aufbereitung derartigen Gewebes besonders geeignet, jedoch nicht auf diese Anwendung beschränkt. Die vorliegende Erfindung ist auch für die Aufbereitung von Blutgefäßen, Hautgewebe, Bändern oder dergleichen anwendbar.

Es gibt grundsätzlich zwei verschiedene Arten von Herzklappenprothesen: mechanische Klappen, welche künstlich, zumeist aus mit pyrolytischem Kohlenstoff beschichteten Graphit, hergestellt werden, und biologische Prothesen, häufig aus Herzbeutelgewebe, welches zumeist aus tierischen Quellen (z.B. Schwein oder Rind) stammt. Die aus dem biologischen Gewebe geformte Herzklappe wird zumeist in einem Grundkörper (z.B. ein festes Kunststoffgerüst oder ein selbstexpandierender Stent) befestigt und dieser wird an der Position der natürlichen Klappe implantiert. Die vorliegende Erfindung beschreibt ein Verfahren zur Aufbereitung eines derartigen Gewebes zum Einsatz in einer Herzklappenprothese, die die Funktion einer natürlichen Herzklappe übernimmt.

Das Ausgangsgewebe muss vor der Implantation gründlich gereinigt und aufbereitet werden. Dabei wird das Gewebe möglichst so modifiziert, dass es vom Körper nicht als Fremdgewebe erkannt wird, nicht verkalkt und eine möglichst lange Lebensdauer hat. Im Wesentlichen umfasst ein derartiges Verfahren zur Aufbereitung von Gewebe mehrere Schritte.

Ein möglicher Aufbereitungsschritt ist die sogenannte Dezellularisierung des Gewebes. In diesem Schritt werden Zellmembranen, intrazelluläre Proteine, Zellkerne und andere Zellbestandteile möglichst vollständig aus dem Gewebe entfernt, um eine möglichst reine extrazelluläre Matrix zu erhalten. Im Gewebe verbleibende Zellen und Zellbestandteile stellen insbesondere eine mögliche Ursache für eine unerwünschte Kalzifizierung des biologischen Implantatmaterials dar. Die Dezellularisierung soll dabei möglichst so schonend durchgeführt werden, dass die Struktur der extrazellulären Matrix und die Kollagenfasern in der extrazellulären Matrix möglichst unbeeinflusst bleiben, während andererseits alle darin enthaltenen Zellen und Zellbestandteile aus dem Gewebe entfernt werden.

Ein weiterer möglicher Aufbereitungsschritt besteht in einer Vernetzung der extrazellulären Matrix, insbesondere der Kollagenfasern, des Gewebes. Nach der Dezellularisierung sind möglichst alle Zellbestandteile aus dem Gewebe entfernt und das biologische Material besteht ausschließlich aus extrazellulärer Matrix. Bei Herzbeutelgewebe wird die extrazelluläre Matrix überwiegend aus Kollagenfasern gebildet. Um ein biologisches Material mit möglichst optimalen mechanischen Eigenschaften zu erreichen und Abwehrreaktionen des empfangenden Körpers zu verhindern, werden die Kollagenfasern mittels eines geeigneten Vernetzungsmittels durch den Einbau chemischer Bindungen quervernetzt. Das Vernetzungsmittel bindet an freie Aminogruppen der Kollagenfasern an und bildet chemisch stabile Verbindungen zwischen Kollagenfasern. So entsteht aus den dreidimensional angeordneten Kollagenfasern ein langzeitstabiles biologisches Material, welches zudem nicht mehr als biologisches Fremdmaterial erkannt wird. Durch die dreidimensionale Vernetzung bzw. Verknüpfung der einzelnen Kollagenfasern über das Vernetzungsmittel werden die Stabilität und die Beanspruchbarkeit des Gewebes deutlich erhöht. Dies ist insbesondere bei einem Einsatz als Gewebe einer Herzklappe entscheidend, wo das Gewebe im Sekundentakt als Klappe öffnen und schließen soll.

Das derart behandelte Gewebe wird an einen Grundkörper befestigt, zumeist durch Vernähen. Der Grundkörper oder das Grundgerüst ist durch chirurgische Techniken implantierbar. Alternativ ist das Grundgerüst selbst expandierend oder mit Hilfe eines Ballons expandierbar, so dass die Herzklappenprothese im komprimierten Zustand mittels eines Katheters an den Implantationsort geführt und innerhalb der natürlichen Klappe implantiert werden kann.

Nach dem Stand der Technik werden derartige mittels Katheter implantierbare Herzklappenprothesen in einer Lagerlösung, entsprechend im feuchten Zustand, aufbewahrt. Die Lagerlösung dient dabei der sterilen Stabilisierung des biologischen Gewebes. Für die Implantation muss die Herzklappenprothese dann im Operationssaal der Lagerlösung entnommen und nach mehreren Spülvorgängen auf dem Katheter montiert werden. Diese Montage der Herzklappenprothese erst im Operationssaal ist umständlich und arbeitsaufwändig. Zusätzlich hängt die korrekte Durchführung der Montage von den Fähigkeiten des jeweiligen Operationsteams ab.

Entsprechend gibt es im Stand der Technik Ansätze, derartiges biologisches Gewebe zu trocknen, im trockenen Zustand zu verarbeiten und zu sterilisieren. Dies würde es ermöglichen ein Gesamtsystem aus Katheter und vormontierter Herzklappenprothese zu sterilisieren und steril verpackt und vormontiert zu versenden.

In WO 99/66967 und in US 2010/030340 werden Verfahren zur Aufbereitung von biologischem Gewebe beschrieben.

Ein Verfahren zur Präparation einer Herzklappenprothese unter der Verarbeitung von getrocknetem, biologischem Material wird in US 8 105 375 offenbart. Nach dem dort offenbarten Verfahren wird das biologische Gewebe nach einer Fixierung oder Vernetzung mit einer Aldehyd enthaltenden Lösung (z.B. Glutaraldehyd- oder Formaldehydlösung) vor der Trocknung mit mindestens einer wässrigen Lösung behandelt, die mindestens einen biokompatiblen und nicht-flüchtigen Stabilisator enthält. Als Stabilisatoren werden dabei hydrophile Kohlenwasserstoffe mit einer Vielzahl von Hydroxylgruppen offenbart und beispielhaft wasserlösliche Zuckeralkohole wie Glycerin, Ethylenglykol oder Polyethylenglykol genannt.

Der vorliegenden Erfindung liegt die Aufgabe zugrunde, ein Verfahren zur Aufbereitung von biologischem Gewebe zur trockenen Verwendung in einem Implantat und ein Implantat derart auszugestalten, dass nicht nur ein hohes Maß an Formstabilität sondern auch ein gezielter Schutz der Gewebestruktur bei einem gleichzeitig geringeren Kalzifizierungsrisiko erreicht wird.

Die gestellte Aufgabe wird verfahrensseitig durch die Merkmalskombination des Anspruches 1 und vorrichtungsseitig durch die Merkmalskombination des Anspruches 10 gelöst.

Erfindungsgemäß wird das Gewebe vor der Trocknung und nach einer Vorbehandlung zur Vernetzung mit einer Aldehyd-haltigen Lösung, insbesondere einer Glutaraldehyd- oder Formaldehyd-haltigen Lösung, einem Form- und Strukturstabilisierungsschritt unterzogen, bei dem das Gewebe einer ersten Lösung ausgesetzt wird, die Polyethylenglykol enthält, und einer zweiten Lösung ausgesetzt wird, die Glycerin enthält, wobei das Gewebe entweder den beiden Lösungen in beliebiger Reihenfolge nacheinander oder gleichzeitig als erstes Lösungsgemisch ausgesetzt wird.

Durch die gezielte Auswahl und Kombination von Polyethylenglykol und Glycerin gelingt überraschenderweise ein gezielter Schutz der Struktur des biologischen Gewebes. Bei der gezielten Kombination von Polyethylenglykol und Glycerin wird nicht nur wie im Stand der Technik eine makroskopische Formstabilität des behandelten biologischen Gewebes bei der Trocknung erreicht, sondern werden die mikroskopischen Gewebestrukturen durch die Stabilisation der Wasserstoffbrücken geschützt und erhalten. Zusätzlich wird durch die Kombination ein spezifischer Schutz des zu trocknenden biologischen Gewebes erreicht. Glycerin und Polyethylenglykol durchdringen das Gewebe und stabilisieren die Struktur. Polyethylenglykol lagert sich zusätzlich konzentriert an der Oberfläche des Gewebes an und schirmt dieses vor äußeren Einflüssen ab. Des Weiteren sorgt die gezielte Verwendung von Polyethylenglykol in Kombination mit Glycerin für eine deutliche Reduktion des Risikos einer Kalzifizierung des implantierten Gewebes.

Gemäß einer besonders bevorzugten Ausführungsform der Erfindung wird das Gewebe vor der Trocknung noch mindestens einer dritten Lösung ausgesetzt, die jeweils Polyethylenglykol mit einem anderen mittleren Molekulargewicht als die vorhergehende Lösung enthält, wobei das Gewebe entweder den Lösungen nacheinander in beliebiger Reihenfolge oder gleichzeitig als Lösungsgemisch ausgesetzt wird. Bevorzugt ist das mittlere Molekulargewicht des Polyethylenglykols der dritten Lösung höher als das mittlere Molekulargewicht des Polyethylenglykols der ersten Lösung. In dieser Ausführungsform der Erfindung können die Lösungen beliebig kombiniert werden. Dies schließt sowohl die Verwendung von drei separaten Lösung, einer separaten Lösung und einem Lösungsgemisch als auch die Verwendung von nur einem Lösungsgemisch enthaltend alle drei Lösungen ein. Ebenso kann es in einigen Ausgestaltungen von Vorteil sein, weitere zusätzliche Polyethylenglykol enthaltende Lösungen zu verwenden.

In dieser bevorzugten Ausführungsform kommen die Vorteile der Erfindung besonders zum Tragen. Diese Ausfuhrungsform basiert auf der Erkenntnis der ErfinderInnen, dass die Eindringtiefe von Polyethylenglykol in das biologische Gewebe vom Molekulargewicht abhängt. Dies liegt mutmaßlich an der sich mit dem Molekulargewicht ändernden Viskosität. Die Verwendung von einer ersten Polyethylenglykol enthaltenden Lösung und einer dritten Lösung, die Polyethylenglykol mit einem anderen mittleren Molekulargewicht als die erste Lösung enthält, ermöglicht eine Stabilisationswirkungen in unterschiedlichen Gewebetiefen. In dieser Ausgestaltung der Erfindung gelingt es entsprechend insbesondere die mikroskopischen Gewebestrukturen zu erhalten und zu stabilisieren.

Bevorzugt enthält die erste Lösung Polyethylenglykol mit einem mittleren Molekulargewicht zwischen 200 g/mol und 6000 g/mol, insbesondere zwischen 200 g/mol und 1000 g/mol.

Gemäß einer besonders bevorzugten Ausgestaltung der Erfindung enthält die erste Lösung Polyethylenglykol mit einem mittleren Molekulargewicht zwischen 400 g/mol und 600g/mol.

In einer weiteren Ausgestaltung der Erfindung enthält die dritte Lösung Polyethylenglykol mit einem mittleren Molekulargewicht zwischen 1000 g/mol und 6000 g/mol, insbesondere mit einem mittleren Molekulargewicht von 4000-6000 g/mol.

Gemäß einer bevorzugten Ausgestaltung der Erfindung erfolgt die Aufbereitung des biologischen Gewebes entweder als Behandlung mit einem ersten Lösungsgemisch, bestehend aus einer ersten Lösung enthaltend Polyethylenglykol mit einem mittleren Molekulargewicht zwischen 400 g/mol und 600 g/mol und einer zweiten Lösung enthaltend Glycerin, oder als Behandlung mit einem zweiten Lösungsgemisch, bestehend aus einer ersten Lösung enthaltend Polyethylenglykol mit einem mittleren Molekulargewicht zwischen 200 g/mol und 1000 g/mol, einer zweiten Lösung enthaltend Glycerin und einer dritten Lösung enthaltend Polyethylenglykol mit einem mittleren Molekulargewicht zwischen 1000 g/mol und 6000 g/mol, insbesondere mit einem mittleren Molekulargewicht von 4000-6000 g/mol.

In einer alternativen Ausgestaltung der Erfindung wird das biologische Gewebe erst einer ersten Lösung enthaltend Polyethylenglykol mit einem mittleren Molekulargewicht zwischen 200 g/mol und 6000 g/mol und anschließend einer zweiten Lösung enthaltend Glycerin ausgesetzt.

In einer weiteren Ausgestaltung der Erfindung wird das biologische Gewebe erst einer dritten Lösung enthaltend Polyethylenglykol mit einem mittleren Molekulargewicht zwischen 1000 g/mol und 6000 g/mol, insbesondere mit einem mittleren Molekulargewicht von 4000-6000 g/mol, anschließend einer ersten Lösung enthaltend Polyethylenglykol mit einem mittleren Molekulargewicht zwischen 200 g/mol und 1000 g/mol gefolgt von einer zweiten Lösung, die Glycerin enthält, ausgesetzt.

Als bevorzugt hat sich eine Ausgestaltung des Verfahrens erwiesen, bei der das biologische Gewebe erst einer Behandlung mit der zweiten Glycerin enthaltenden Lösung, anschließend einer Behandlung mit der ersten Polyethylenglykol mit einem niedrigen mittleren Molekulargewicht im Bereich von 200 g/mol bis 400 g/mol enthaltender Lösung gefolgt von einer Behandlung mit einer weiteren ersten Lösung, enthaltend Polyethylenglykol mit einem leicht erhöhten mittleren Molekulargewicht, unterzogen wird. In dieser Ausgestaltung dringt das Glycerin der zweiten Lösung erst tief in das Gewebe ein, das Polyethylenglykol der ersten Lösung (z.B. eine Lösung enthaltend Polyethylenglykol mit einem mittleren Molekulargewicht von 200 g/mol) dringt in die oberflächennahen Bereiche ein und das Polyethylenglykol der dritten Lösung (z. B. eine Lösung enthaltend Polyethylenglykol mit einem mittleren Molekulargewicht von 400 g/mol) bewirkt eine Versiegelung der Oberfläche. In dieser Ausgestaltung können die beiden Polyethylenglykol enthaltenden Lösungen auch gleich sein.

Zweckmäßigerweise wird das Gewebe der ersten oder der dritten Lösung oder dem ersten bzw. zweiten Lösungsgemisch für 1 bis 12 Stunden, bevorzugt für 2 bis 6 Stunden, besonders bevorzugt für 2 Stunden, ausgesetzt. Dabei liegt vorteilhafterweise Polyethylenglykol in der ersten oder dritten Lösung oder dem ersten bzw. zweiten Lösungsgemisch in einer Konzentration von 5 Vol-% bis 60 Vol-%, bevorzugt von 10 Vol-% bis 40 Vol-%, vor. Glycerin ist zweckmäßigerweise in der zweiten Lösung oder dem ersten bzw. zweiten Lösungsgemisch in einer Konzentration von 5 Vol-% bis 50 Vol-%, bevorzugt 5 Vol-% bis 30 Vol-%.

Im Rahmen dieser Anmeldung bezieht sich die Angabe Vol-% auf einen Volumenprozentanteil. Eine 100 ml Lösung mit 5 Vol-% Polyethylenglykol enthält entsprechend 5 ml reines Polyethylenglykol. Die Angabe Gew-% bezieht sich im Rahmen dieser Anmeldung auf einen Gewichtsprozentanteil. Ein 100 g Lösung mit 0.9 Gew-% Natriumchlorid enthält entsprechend 0.9 g Natriumchlorid.

Bevorzugt wurde das biologische Gewebe einer Vorbehandlung unterzogen, die eine optionale Dezellularisierung mit einem geeigneten Detergenz, bevorzugt mit einer Surfactin und Desoxycholsäure enthaltenden Lösung, und einer anschließenden Vernetzung, bevorzugt mit einer Glutaraldehyd enthaltenden Lösung, umfasst.

Das in dieser Anmeldung beschriebene Verfahren ist für die Aufbereitung von nativen, vernetzten, dezellularisierten oder nicht dezellularisierten Gewebe geeignet.

Vorrichtungsseitig wird die gestellte Aufgabe durch ein Implantat gelöst, welche biologisches Gewebe aufweist, das dem erfindungsgemäßen Verfahren zur Aufbereitung unterzogen und getrocknet wurde.

Die Trocknung des Gewebes wird dabei bevorzugt so ausgestaltet, dass ein langsamer und schonender Entzug des Wassers im flüssigen Zustand aus dem Gewebe gewährleistet ist. Dies gelingt vorteilhafterweise durch die kontrollierte Reduktion der Umgebungsfeuchte des biologischen Gewebes in einem Exsikkator oder einem Klimaschrank unter kontrollierter Einstellung der Parameter der Umgebungsatmosphäre des biologischen Gewebes.

Bevorzugt ist das Implantat eine Herzklappenprothese, welche eine künstliche Herzklappe aus biologischen Gewebe und/oder eine Abdichtung aus biologischen Gewebe aufweist, welches an einen expandierbaren oder selbstexpandierenden und per Katheter implantierbaren Grundgerüst befestigt, bevorzugt vernäht, ist.

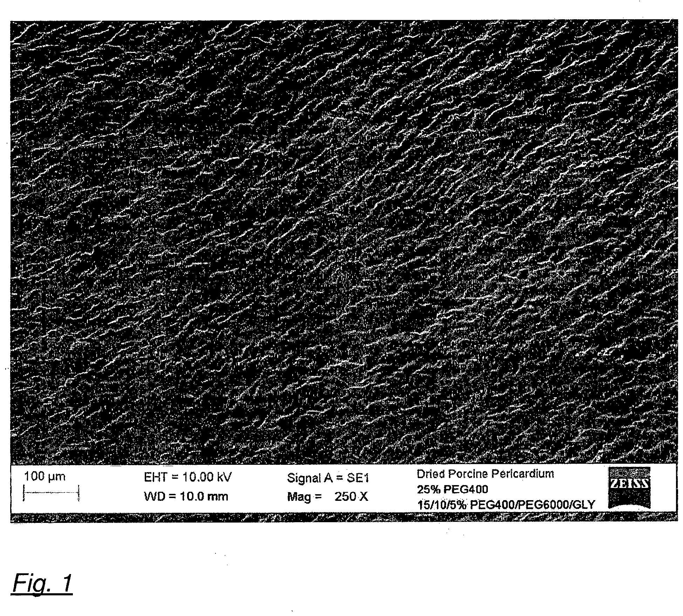

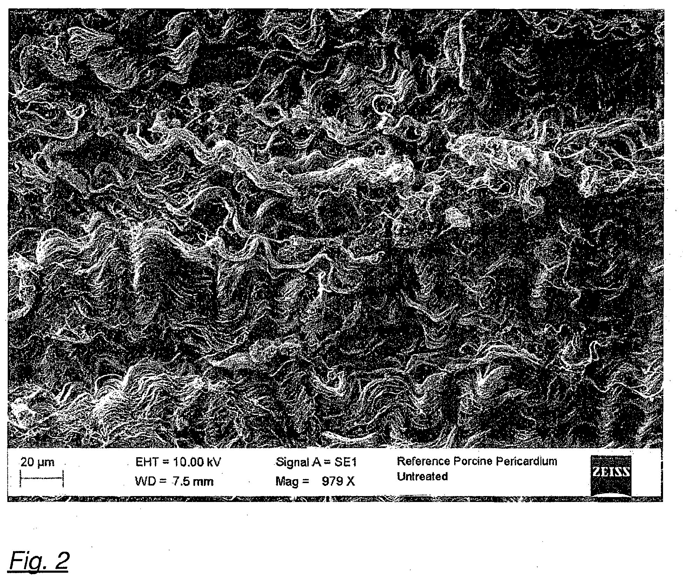

Im Folgenden soll die Erfindung anhand von drei Ausführungsbeispielen und eines in den Figuren 1 und 2 dargestellten Vergleiches eines getrockneten biologischen Gewebes nach einem Ausführungsbeispiel der Erfindung mit einem Beispiel nach dem Stand der Technik näher erläutert werden.

### Beispiel 1:

Beispiel 1 offenbart eine Ausgestaltung des erfindungsgemäßen Verfahrens zur Aufbereitung von porcinem Perikardgewebe mit anschließender Trocknung.

Zunächst wird im Schlachthof einem Schwein ein Herzbeutel frisch entnommen und für 2 Stunden bei einer Temperatur von 4 °C in einer Penicillin und/oder Streptomycin enthaltenden 0.9 Gew-% Natriumchloridlösung gelagert. Im nächsten Schritt werden Fett und Bindegewebe feucht (0.9 Gew% Natriumchloridlösung) vom Perikardgewebe abgetrennt und das Perikardgewebe zugeschnitten.

Anschließend wird das Gewebe gespült (100 ml 0.9 Gew-% Natriumchloridlösung unter leichter Bewegung), vernetzt (48 Stunden in 100 ml 0.6 Gew-% Glutaraldehydlösung (Glutaraldehyde in gepufferter Salzlösung bei 4-8°C (DPBS-Lösung, Fa. Lonza; DPBS w/o Ca++/Mg++; Art.-Nr. 17-512)), wobei diese Lösung danach über 14 Tage lang bei Raumtemperatur einwirkt und alle 48 Stunden gegen eine analoge, frische Lösung ausgetauscht wird) und anschließend erneut gespült (Spülung für 10 min in 100 ml 0.9 Gew-% Natriumchloridlösung bei 37°C unter leichter Bewegung 6-mal wiederholen).

Das derart behandelte vernetzte biologische Gewebe wird anschließend einer Ausgestaltung des erfindungsgemäßen Form- und Strukturstabilisierungsschritts unterzogen.

In dieser Ausgestaltung wird das biologische Gewebe einer ersten Spülung für 10 min in 100 ml wässriger 25 Vol-% Polyethylenglykollösung (enthaltend Polyethylenglykol mit einem mittleren Molekulargewicht von 400 g/mol) bei 37 °C mit 3 Wiederholungen unterzogen.

Anschließend wird es einem zweiten Lösungsgemisch für 2 Stunden bei einer Temperatur von 37 °C ausgesetzt. Das zweite Lösungsgemisch besteht in dieser Ausgestaltung der Erfindung aus einer wässrigen Lösung enthaltend Polyethylenglykol mit einem mittleren Molekulargewicht von 400 g/mol in einer Konzentration von 15 Vol-%, Polyethylenglykol mit einem mittleren Molekulargewicht von 6000 g/mol in einer Konzentration von 10 Vol-% und Glycerin mit einer Konzentration von 5 Vol-%.

Das derart stabilisierte biologische Gewebe wird anschließend in einem Exsikkator mit einem Silicagel als Trocknungsmittel für 24 Stunden getrocknet. Das so erhaltene getrocknete, biologische Gewebe kann entweder weiter verarbeitet oder im Exsikkator gelagert werden.

Unter einem Exsikkator wird im Rahmen dieser Anmeldung ein abgeschlossenes Gefäß verstanden, welches ein aktives Trocknungsmittel enthält und im Inneren eine minimale Luftfeuchte aufweist.

Alternativ kann die Trocknung auch in einem Klimaschrank mit einstellbarer Temperatur und Luftfeuchte erfolgen.

### Beispiel 2:

Analog zum Beispiel 1 wird ein Herzbeutel vom Schwein entnommen, für 2 Stunden bei einer Temperatur von 4 °C in einer Penicillin und/oder Streptomycin enthaltenden 0.9 Gew-% Natriumchloridlösung gelagert, unter Entfernung von Fett und Bindegewebe feucht (0.9 Gew- % Natriumchloridlösung) präpariert, zugeschnitten und anschließend mit 100 ml 0.9 Gew-% Natriumchloridlösung unter leichter Bewegung gespült.

Das derart gewonnene Perikardgewebe wird anschließend einer schonenden Dezellularisierung und anschließender Vernetzung unterzogen. Dabei sind die folgenden Schritte umfasst:
- Dezellularisierung in 100 ml Surfactin/Desoxycholsäure-Lösung (0.06 Gew-% Surfactin und 0.5 Gew-% Desoxycholsäure in 0.9 Gew-% Natriumchloridlösung) für 20 Stunden bei 37°C,
- Spülen mit 100 ml 0.9 Gew-% Natriumchloridlösung (6-mal unter leichter Bewegung für 10 min)
- Behandlung mit einer DNase-Lösung für 12 Stunden bei 37 °C
- Spülen mit 100 ml 0.9 Gew-% Natriumchloridlösung (8-mal unter leichter Bewegung für 10 min)
- Spülen mit 100 ml 70 Vol-% Ethanollösung (einmal für 10 min)
- Spülen mit 100 ml 0.9 Gew-% Natriumchloridlösung
- Vernetzung mit Glutaraldehyd (48 Stunden in 100 ml 0.6 Gew-% Glutaraldehydlösung (Glutaraldehyde in gepufferter Salzlösung bei 4-8°C (DPBS-Lösung, Fa. Lonza; DPBS w/o Ca++/Mg++; Art.-Nr. 17-512)), wobei diese Lösung danach über 14 Tage lang bei Raumtemperatur einwirkt und alle 48 Stunden gegen eine analoge, frische Lösung ausgetauscht wird)
- Spülen mit 100 ml 0.9 Gew-% Natriumchloridlösung (6-mal unter leichter Bewegung für 10 min)

Das derart entstandene dezellularisierte und vernetzte Perikardgewebe wird in dieser Ausgestaltung der Erfindung in drei Schritten stabilisiert. Zuerst wird das Gewebe mit 100 ml wässriger 25 Vol-% Polyethylenglykollösung (enthaltend Polyethylenglykol mit einem mittleren Molekulargewicht von 400 g/mol bei 37 °C, 3-mal für 10 min) gespült. Anschließend wird das Gewebe einer wässrigen Lösung enthaltend 20 Vol-% Polyethylenglykol mit einem mittleren Molekulargewicht von 400 g/mol und 10 Vol-% Glycerin für 2 Stunden bei 37 °C unter leichter Bewegung ausgesetzt. Anschließend erfolgt unter leichter Bewegung bei gleicher Temperatur für 2 Stunden eine Behandlung mit einer wässrigen Lösung enthaltend 20 Vol-% Polyethylenglykol mit einem mittleren Molekulargewicht von 6000 g/mol und 10 Vol-% Glycerin.

Das derart stabilisierte biologische Gewebe wird anschließend in einem Exsikkator mit einem Silicagel als Trocknungsmittel für 24 Stunden getrocknet und anschließend weiterverarbeitet.

### Beispiel 3:

In der Ausgestaltung nach Beispiel 3 wird bereits vernetztes porcines Perikardgewebe mit folgendem Verfahren aufbereitet (stabilisiert und getrocknet):
- Spülen mit 100 ml 0.9 Gew-% Natriumchloridlösung (6-mal unter leichter Bewegung für 10 min bei Raumtemperatur)
- Spülen mit 100 ml wässriger 40 Vol-% Glycerinlösung (3-mal unter leichter Bewegung für 20 min bei 37°C)
- Einlegen des Perikardgewebes in wässrige Lösung enthaltend 30 Vol-% Polyethylenglykol mit einem mittleren Molekulargewicht von 400 g/mol und 10 Vol-% Glycerin für 2 Stunden bei 37 °C unter leichter Bewegung
- Einlegen des Perikardgewebes in wässrige Lösung enthaltend 30 Vol-% Polyethylenglykol mit einem mittleren Molekulargewicht von 6000 g/mol und 10 Vol-% Glycerin für 2 Stunden bei 37 °C unter leichter Bewegung
- Trocknen im Exsikkator mit Silicagel als Trocknungsmittel für 24 Stunden.

Des Weiteren ist mit den Figuren 1 und 2 ein Vergleich des Ausführungsbeispiels 1 der Erfindung mit dem Stand der Technik dargestellt.

Es zeigen:
- Fig. 1: eine elektronenmikroskopische Darstellung eines porcinen Perikardgewebes gemäß Beispiel 1 und
- Fig. 2: ein nicht behandeltes vernetztes Perikard als Referenz.

Fig. 1 zeigt ein porcines Perikardgewebe im getrockneten Zustand nach einer Behandlung gemäß Beispiel 1 der Erfindung. Anhand der elektronenmikroskopischen Aufnahme ist eine Versiegelung und Stabilisierung der Oberflächenstruktur durch die Behandlung mit den Stabilisatoren sehr gut zu erkennen. Das in Fig. 2 gezeigte, unbehandelte Referenzgewebe weist freiliegende Kollagenstrukturen auf, die unversiegelt vorliegen.

## Patentansprüche

1. Verfahren zur Aufbereitung von biologischem Gewebe für die Verwendung als Bestandteil eines Implantats, insbesondere zur Verwendung als Bestandteil einer Herzklappenprothese, **dadurch gekennzeichnet, dass** das Gewebe in einem Vorbehandlungsschritt einer Vernetzung mit einer Aldehyd-haltigen Lösung, insbesondere einer Glutaraldehyd- oder Formaldehyd-haltigen Lösung, unterzogen wird und vor der Trocknung einem Form- und Strukturstabilisierungsschritt unterzogen wird, bei dem das Gewebe einer ersten Lösung ausgesetzt wird, die Polyethylenglykol enthält, und einer zweiten Lösung ausgesetzt wird, die Glycerin enthält, wobei das Gewebe entweder den beiden Lösungen in beliebiger Reihenfolge nacheinander oder gleichzeitig als erstes Lösungsgemisch ausgesetzt wird.

2. Verfahren nach Anspruch 1, **dadurch gekennzeichnet, dass** das Gewebe vor der Trocknung einer dritten Lösung ausgesetzt wird, die Polyethylenglykol mit einem anderen mittleren Molekulargewicht als die erste Lösung enthält, wobei das Gewebe entweder den Lösungen nacheinander in beliebiger Reihenfolge oder gleichzeitig als zweites Lösungsgemisch ausgesetzt wird.

3. Verfahren nach Anspruch 1 oder 2, **dadurch gekennzeichnet, dass** die erste Lösung Polyethylenglykol mit einem mittleren Molekulargewicht zwischen 200 g/mol und 6000 g/mol enthält.

4. Verfahren nach einem der vorangegangenen Ansprüche, **dadurch gekennzeichnet, dass** die erste und/oder dritte Lösung Polyethylenglykol mit einem mittleren Molekulargewicht zwischen 200 g/mol und 1000 g/mol enthält.

5. Verfahren nach einem der Ansprüche 2 bis 4, **dadurch gekennzeichnet, dass** die dritte Lösung Polyethylenglykol mit einem mittleren bis höheren Molekulargewicht zwischen 1000 g/mol und 6000 g/mol, insbesondere mit einem mittleren Molekulargewicht von 4000-6000 g/mol, enthält.

6. Verfahren nach einem der vorangegangenen Ansprüche, **dadurch gekennzeichnet, dass** das Gewebe der ersten oder der dritten Lösung oder dem ersten bzw. zweiten Lösungsgemisch für 1 bis 12 Stunden, bevorzugt für 2 bis 6 Stunden, besonders bevorzugt für 2 Stunden, ausgesetzt wird.

7. Verfahren nach einem der vorangegangenen Ansprüche, **dadurch gekennzeichnet, dass** Polyethylenglykol in der ersten oder dritten Lösung oder dem ersten bzw. zweiten Lösungsgemisch in einer Konzentration von 5 Vol-% bis 60 Vol-%, bevorzugt von 10 Vol-% bis 40 Vol-%, vorliegt.

8. Verfahren nach einem der vorangegangenen Ansprüche, **dadurch gekennzeichnet, dass** Glycerin in der zweiten Lösung oder dem ersten bzw. zweiten Lösungsgemisch in einer Konzentration von 5 Vol-% bis 50 Vol-%, bevorzugt 5 Vol-% bis 30 Vol-%, vorliegt.

9. Verfahren nach einem der vorangegangen Ansprüche, **dadurch gekennzeichnet, dass** das biologische Gewebe einer Vorbehandlung unterzogen wurde, die eine optionale Dezellularisierung mit einem geeigneten Detergenz, bevorzugt mit einer Surfactin und Desoxycholsäure enthaltenden Lösung, und eine Vernetzung, bevorzugt mit einer Glutaraldehyd enthaltenden Lösung, umfasst.

10. Implantat, **dadurch gekennzeichnet, dass** es biologisches Gewebe aufweist, welches einem Verfahren zur Aufbereitung nach den Ansprüchen 1 bis 9 unterzogen und getrocknet wurde.

11. Implantat nach Anspruch 10, **dadurch gekennzeichnet, dass** das Implantat eine Herzklappenprothese ist, welche eine künstliche Herzklappe aus biologischen Gewebe und/oder eine Abdichtung aus biologischen Gewebe aufweist, welches an einen expandierbaren oder selbstexpandierenden und per Katheter implantierbaren Grundkörper befestigt, bevorzugt vernäht, ist.

## Claims

1. A method for preparing biological tissue for use as a component of an implant, in particular for use as part of a heart valve prosthesis, **characterised in that** the tissue is subjected in a pretreatment step to a cross-linking with an aldehyde-containing solution, in particular a glutaraldehyde- or formaldehyde-containing solution, and, before drying, is subjected to a dimensional and structural stabilisation step in which the tissue is exposed to a first solution, which contains polyethylene glycol, and is exposed a second solution, which contains glycerol, wherein the tissue is exposed either to the two solutions one after the other in any order, or is exposed to both solutions simultaneously, as a first solution mixture.

2. The method according to claim 1, **characterised in that**, prior to the drying, the tissue is exposed to a third solution, which contains polyethylene glycol having a mean molecular weight that differs from that of the first solution, wherein the tissue is either exposed to the solutions one after the other in any order or simultaneously as a second solution mixture.

3. The method according to claim 1 or 2, **characterised in that** the first solution contains polyethylene glycol having a mean molecular weight between 200 g/mol and 6000 g/mol.

4. The method according to any one of the preceding claims, **characterised in that** the first and/or third solution contains polyethylene glycol having a mean molecular weight between 200 g/mol and 1000 g/mol.

5. The method according to any one of claims 2 to 4, **characterised in that** the third solution contains polyethylene glycol having a mean to higher molecular weight between 1000 g/mol and 600 g/mol, in particular having a mean molecular weight of 4000-6000 g/mol.

6. The method according to any one of the preceding claims, **characterised in that** the tissue is exposed to the first or the third solution or to the first or the second solution mixture for 1 to 12 hours, preferably for 2 to 6 hours, particularly preferably for 2 hours.

7. The method according to any one of the preceding claims, **characterised in that** the polyethylene glycol is present in the first or the third solution or in the first or second solution mixture in a concentration of 5 vol % to 60 vol %, preferably of 10 vol % to 40 vol %.

8. The method according to any one of the preceding claims, **characterised in that** glycerol is contained in the second solution or in the first or second solution mixture in a concentration of 5 vol % to 50 vol %, preferably 5 vol % to 30 vol %.

9. The method according to any one of the preceding claims, **characterised in that** the biological tissue has been subjected to a pretreatment that comprises an optional decellularisation with a suitable detergent, preferably a solution containing surfactin and deoxycholic acid, and a cross-linking, preferably with a glutaraldehyde-containing solution.

10. An implant, **characterised in that** it comprises biological tissue which has been subjected to a preparation method according to any one of claims 1 to 9 and has been dried.

11. The implant according to claim 10, **characterised in that** the implant is a heart valve prosthesis, which comprises an artificial heart valve made of biological tissue and/or a seal made of biological tissue, which is secured on an expandable or self-expanding support body, which is implantable by means of a catheter, preferably being secured thereon via suturing.

## Revendications

1. Procédé de préparation de tissu biologique pour l'utilisation en tant que composant d'un implant, en particulier pour l'utilisation en tant que composant d'une prothèse de valve cardiaque, **caractérisé en ce que** le tissu est soumis à une réticulation avec une solution contenant un aldéhyde dans une étape de prétraitement, en particulier une solution contenant du glutaraldéhyde ou du formaldéhyde, et est soumis à une étape de stabilisation de la forme et de la structure avant le séchage, dans laquelle le tissu est exposé à une première solution qui contient du polyéthylène glycol, et est exposé à une deuxième solution qui contient de la glycérine, où le tissu est exposé soit aux deux solutions dans un ordre quelconque l'une après l'autre, soit simultanément sous la forme d'un premier mélange de solutions.

2. Procédé selon la revendication 1, **caractérisé en ce que** le tissu est exposé avant le séchage à une troisième solution qui contient du polyéthylène glycol avec un autre poids moléculaire moyen que la première solution, où le tissu est exposé aux solutions soit l'une après l'autre dans un ordre quelconque, soit simultanément sous la forme d'un deuxième mélange de solutions.

3. Procédé selon la revendication 1 ou 2, **caractérisé en ce que** la première solution contient du polyéthylène glycol avec un poids moléculaire moyen entre 200 g/mol et 6000 g/mol.

4. Procédé selon l'une des revendications précédentes, **caractérisé en ce que** la première et/ou la troisième solution contient du polyéthylène glycol avec un poids moléculaire moyen entre 200 g/mol et 1000 g/mol.

5. Procédé selon l'une des revendications 2 à 4, **caractérisé en ce que** la troisième solution contient du polyéthylène glycol avec un poids moléculaire moyen à élevé entre 1000 g/mol et 6000 g/mol, notamment avec un poids moléculaire moyen de 4000 à 6000 g/mol.

6. Procédé selon l'une des revendications précédentes, **caractérisé en ce que** le tissu est exposé à la première ou à la troisième solution, ou au premier, respectivement, au deuxième mélange de solutions pour 1 à 12 heures, de préférence, pour 2 à 6 heures, de manière particulièrement préférée, pour 2 heures.

7. Procédé selon l'une des revendications précédentes, **caractérisé en ce que** du polyéthylène glycol est présent dans la première ou dans la troisième solution, ou dans le premier, respectivement, le deuxième mélange de solutions à une concentration de 5 % en volume à 60 % en volume, de préférence, de 10 % en volume à 40 % en volume.

8. Procédé selon l'une des revendications précédentes, **caractérisé en ce que** de la glycérine est présente dans la deuxième solution, ou dans le premier, respectivement, le deuxième mélange de solutions à une concentration de 5 % en volume à 50 % en volume, de préférence, de 5 % en volume à 30 % en volume.

9. Procédé selon l'une des revendications précédentes, **caractérisé en ce que** le tissu biologique a été soumis à un prétraitement qui comprend une décellularisation éventuelle avec un détergent approprié, de préférence avec une solution contenant de la surfactine et de l'acide désoxycholique, et à une réticulation de préférence avec une solution contenant du glutaraldéhyde.

10. Implant **caractérisé en ce qu'**il présente du tissu biologique, lequel a été soumis à un procédé de préparation selon les revendications 1 à 9 et a été séché.

11. Implant selon la revendication 10, **caractérisé en ce que** l'implant est une prothèse de valve cardiaque, laquelle présente une valve cardiaque synthétique à base d'un tissu biologique et/ou un joint à base de tissu biologique, lequel est fixé, de préférence, cousu sur un corps de base expansible ou auto-expansible et implantable par un cathéter.
